# DEMANDE DE BREVET EUROPEEN

(11) **EP 3 875 582 A1**
(43) Date de publication de la demande: **08.09.2021**
(21) Numéro de dépôt: 20305214.7
(22) Date de dépôt: 02.03.2020
(51) Int. Cl.: C12N 5/077

(54) **PROCEDE DE DIFFERENCIATION DE CELLULES SOUCHES PLURIPOTENTES EN FIBROBLASTES DE TISSUS CONJONCTIFS SOUS-JACENTS D'UN EPITHELIUM**

(71) Demandeur: URGO RECHERCHE INNOVATION ET DEVELOPPEMENT, 21300 Chenôve (FR); Centre d'Etude des Cellules Souches (CECS), 91100 Corbeil Essonnes (FR); Universite d'Evry Val d'Essonne, 91000 Evry (FR)
(72) Inventeur: LEMAITRE, Gilles, 91080 Evry-Courcouronnes (FR); BALDESCHI, Christine, 91360 Villemoisson Sur Orge (FR); DOMINGUES, Sophie, 91100 Corbeil-Essonnes (FR)
(74) Mandataire: Plasseraud IP

(57) **Abrégé**

L'invention concerne un procédé de différenciation de cellules souches pluripotentes humaines en fibroblastes, caractérisé en ce que les cellules souches pluripotentes humaines sont cultivées sur un système adhérent en présence d'un milieu adapté à la culture des fibroblastes et en l'absence de cellules nourricières.

## Description

La présente invention concerne un procédé de différenciation de cellules souches pluripotentes (Pluripotent Stem Cell ou PSC) (embryonnaires ou cellules souches induites à la pluripotence (iPS)) en fibroblastes de tissus conjonctifs fibreux sous-jacents d'un épithélium.

### Contexte de l'invention

Les cellules souches pluripotentes humaines, qu'elles soient embryonnaires ou induites à la pluripotence, présentent une capacité à proliférer à l'identique (chaque cellule-mère donnant naissance à deux cellules-filles identiques à la première) à l'infini, sans jamais entrer en sénescence comme le font toutes les autres cellules de l'organisme, et la capacité, dans d'autres conditions de culture, à se différencier pour donner naissance à n'importe quelle cellule de l'organisme (ectoderme, endoderme et mésoderme).

Les cellules souches sont importantes en médecine régénérative (source d'intérêt majeur et prometteur pour réussir à fabriquer des organes), la modélisation, notamment de maladies, et le criblage pharmacologique.

Les cellules souches pluripotentes apparaissent comme une alternative possible du fait de leur propriété de prolifération illimitée et leur capacité de différenciation permettant d'obtenir à partir d'un même donneur, tous les types cellulaires d'intérêt en grande quantité. De plus, les cellules souches pluripotentes permettent d'obtenir une population homogène de cellules différenciées, contrairement aux cultures primaires (hétérogénéité, limite en nombre, risque de sénescence).

La présente invention vise une méthode de différenciation de cellules souches pluripotentes humaines en fibroblastes, préférentiellement en fibroblastes de tissus conjonctifs sous-jacents d'un épithélium, encore plus préférentiellement de fibroblastes dermiques, dans des conditions dites « cliniques »., On entend par conditions « cliniques » des conditions où les produits utilisés sont fabriqués selon les Bonnes Pratiques de Fabrication (BPF ou GMP - Good Manufacturing Practices) et peuvent donc être utilisés dans des études cliniques contrairement aux produits de grade « recherche » qui ne peuvent être utilisés que pour la recherche.

Les fibroblastes sont des cellules dites de soutien, présentes notamment dans le tissu conjonctif et responsables de la synthèse de la matrice extracellulaire et des fibres de collagène (substance intercellulaire du tissu conjonctif).

On entend par tissus conjonctifs sous-jacents d'un épithélium, dans le cadre de la présente invention, les tissus conjonctifs denses à prédominance de fibres de collagène, qui assurent un rôle mécanique dans l'organisme, comme le derme, les tendons ou la cornée par exemple.

Les fibroblastes dermiques sont de deux types en fonction de leur localisation dans le derme.

Dans sa partie superficielle au contact de l'épiderme (derme papillaire), le derme est constitué de tissu conjonctif dense fibreux orienté et contient majoritairement des fibroblastes papillaires alors qu'il est constitué de tissu conjonctif dense fibreux non orienté, majoritairement composé de fibroblastes réticulaires dans le derme profond (ou derme réticulaire).

Le procédé selon la présente invention permet d'obtenir préférentiellement des fibroblastes dermiques.

De manière avantageuse, les fibroblastes obtenus selon le procédé de la présente invention sont des fibroblastes dermiques papillaires.

Les normes « BPF » constituent une notion d'assurance qualité établie par la commission européenne (ou américaine) dans le cadre de la fabrication de médicaments à usage humain ou vétérinaire (EUDRALEX en France ou FDA aux Etats-Unis). Elles ont été créées pour limiter les risques de contamination croisée des produits, en insistant sur les pratiques d'hygiène, ainsi que les risques de confusion : étiquetage/identification.

Les principes des BPF imposent l'écriture de modes opératoires et d'instructions permettant des productions de qualité régulière avec une traçabilité conforme. Ils intègrent également les procédés, la qualité du produit et la sécurité du personnel. Les BPF sont actuellement organisées en 3 parties :
- Les bonnes pratiques de fabrication des médicaments à usage humain.
- Les bonnes pratiques de fabrication pour les substances actives utilisées comme matière première dans les médicaments.
- Les documents relatifs aux bonnes pratiques de fabrication donnant les recommandations sur les exigences internationales pour la certification de lots.

Ainsi, pour établir des protocoles de différenciation « cliniques », les matières premières doivent répondre aux mêmes normes BPF. Les produits utilisés sont donc entièrement définis au niveau de leurs compositions, de leurs concentrations, de leurs origines et de leurs niveaux de stérilité.

A ce jour, il n'existe aucun procédé de différenciation en fibroblastes à partir de cellules souches humaines pluripotentes répondant aux normes BPF, permettant d'obtenir des populations cellulaires spécifiques homogènes robustes (qui ne dévient pas, restent les mêmes dans le temps).

Il y a donc un besoin de développer des méthodes d'obtention de populations de fibroblastes humains pouvant être utilisés en thérapie tissulaire ou pour des modèles *in vitro* et *in vivo* de peau humaine. Les cellules souches embryonnaires et les cellules somatiques qui sont génétiquement reprogrammées afin de reproduire toutes les caractéristiques des cellules souches embryonnaires (telles que, par exemple, le "iPS", pour les cellules "souches pluripotentes induites") sont des cellules souches pluripotentes à prolifération extensive et ont par conséquent un grand potentiel dans la recherche et la médecine. Plusieurs tentatives ont donc été décrites dans l'art antérieur pour obtenir des fibroblastes humains à partir de cellules souches pluripotentes.

Notamment, le procédé décrit dans le journal « Stem Cell Research and Therapy » par l'équipe de Shamis et al, en 2011 utilise des cellules souches embryonnaires humaines (hESC) cultivées sur une couche de fibroblastes nourriciers dérivés d'embryon de souris inactivées par un traitement au para-formaldéhyde 4% (appelée « feeder »). (Shamis Y, Hewitt KJ, Carlson MW, Margvelashvilli M, Dong S, Kuo CK, Daheron L, Egles C, Garlick JA. Fibroblasts derived from human embryonic stem cells direct development and repair of 3D human skin equivalents. Stem Cell Res Ther. 2011 Feb 21;2(1):10. doi: 10.1186/scrt51.)

Ceci nécessite de traiter la couche de feeder, par exemple des fibroblastes murins 3T3, par un agent inhibant totalement la prolifération (anti-mitotique) de ces cellules au profit des cellules hPSC. Cependant, les produits utilisés pour bloquer la prolifération des feeders peuvent s'avérer toxiques pour les cellules d'intérêt.

De plus, l'origine des feeders est murine. Afin que ce système puisse être utilisé avec des feeders murins, il faut garantir que ces feeders puissent être éliminés au cours du procédé pour purifier la population de fibroblastes humains. Les feeders étant des fibroblastes également, il n'est pas possible à l'heure actuelle, de séparer les fibroblastes humains des fibroblastes murins.

Le procédé décrit dans "Generation of 3D skin equivalents fully reconstituted from human induced pluripotent stem cells (iPSCs)" de Munenari Itoh, Noriko Umegaki-Arao, Zongyou Guo, Liang Liu, Claire A. Higgins, Angela M. Christiano porte quant à lui sur un système de culture en suspension qui nécessite un traitement à J8 pour passer les cellules en adhérentes.

Par conséquent, il y a donc un besoin d'améliorer les méthodes existantes pour générer et obtenir une population homogène de fibroblastes. De plus, il y a également un besoin d'obtenir des cellules d'une plus grande pureté.

La présente invention propose des méthodes permettant la production de fibroblastes dérivés d'iPSC ou ES nécessaires à des applications cliniques, de recherche ou thérapeutiques indépendantes des EB, qui n'utilisent pas de cellules nourricières, et surtout qui fournissent une population homogène et pure de fibroblastes.

La présente invention vise une méthode de différenciation de cellules souches pluripotentes en fibroblastes, dans des conditions dites « cliniques ».

### Exposé de l'invention

La présente invention concerne un procédé de différenciation de cellules souches pluripotentes humaines d'origine embryonnaire ou induites en fibroblastes, de préférence en fibroblastes du tissu conjonctif sous-jacents d'un épithélium, compatible avec les normes BPF.

Plus particulièrement, la présente invention concerne un procédé de différenciation de fibroblastes à partir de cellules souches pluripotentes humaines, caractérisé en ce que les cellules souches pluripotentes humaines sont cultivées sur un système adhérent en présence d'un milieu adapté à la culture des fibroblastes et en l'absence de cellules nourricières.

Avantageusement, le milieu adapté à la culture des fibroblastes est un milieu chimiquement défini qui ne contient aucun additif d'origine animale non contrôlé aux normes de Bonnes Pratiques de Fabrication (ou BPF).

Par « système adhérent » on entend tout système permettant aux cellules d'adhérer en 2D. Un tel système permet une sélection de cellules différenciées homogènes, contrairement aux systèmes en suspension qui augmentent le risque d'avoir des populations hétérogènes. Il s'agit d'une surface recouverte d'une matrice ou « coating ». De manière préférée, la matrice utilisée dans le procédé selon l'invention est une matrice protéique définie. De manière préférée, la matrice est choisie dans le groupe constitué par Matrigel™, L7 coating™, la laminine et la vitronectine. De manière particulièrement préféré, la matrice est la matrice L7™ commercialisée par la société Lonza sous la référence FP-5020.

Par « hPSC » on entend toutes cellules précurseurs d'origine humaine qui ont la capacité de former n'importe quelle cellule adulte. Ces cellules sont de véritables lignées cellulaires en ce qu'elles : (i) sont capables d'une prolifération extensive *in vitro* dans un état indifférencié ; et (ii) sont capables de se différencier en dérivés des trois couches germinales embryonnaires (endoderme, mésoderme et ectoderme) même après une culture prolongée. Les cellules souches embryonnaires humaines (CSEh, ou hESC, en anglais) sont dérivées d'embryons fécondés qui ont moins d'une semaine (dans le clivage ou stade blastocytaire) ou produites par des moyens artificiels (tels que par transfert nucléaire) qui ont des caractéristiques équivalentes. D'autres hPSC incluent, sans s'y limiter, les cellules progénitrices adultes multipotentes (MAP), les cellules souches pluripotentes induites (iPSC) et les cellules souches du liquide amniotique.

Dans un mode de réalisation préféré, les cellules souches pluripotente humaines sont obtenues par des méthodes qui ne nécessitent pas la destruction d'embryons.

Dans un mode de réalisation, les cellules souches pluripotentes sont des cellules souches embryonnaires humaines (« human embryonic stem cell » ou « hESC »). Il existe de nombreuses lignées de hESC, dont la lignée RC-9 (« Roslin Cells 9 »), qui a été développée en tant que lignée hESC de grade clinique.

Alternativement, les cellules hESC ou les cellules iPSC humaines peuvent être sélectionnées parmi des banques de cellules maîtresses qui peuvent être constituées à des fins thérapeutiques. De manière préférée, les cellules hESC ou iPSC humaines peuvent être sélectionnées pour éviter ou limiter le rejet immunitaire dans la majorité de la population humaine. En règle générale, les cellules hESC ou les cellules iPSC humaines sont homozygotes HLA pour les gènes codant pour les principaux antigènes d'histocompatibilité A, B et DR, ce qui signifie qu'elles ont un profil génétique simple dans le répertoire HLA. Les cellules pourraient servir à créer une banque de cellules souches en tant que source renouvelable de cellules pouvant convenir à la préparation de substituts cutanés humains destinés à être utilisés dans la thérapie cellulaire des pathologies associées à des lésions cutanées (par exemple, plaie, brûlures, irradiation, anomalies épidermiques liées à la maladie). Dans un autre mode de réalisation particulier, les cellules souches pluripotentes humaines peuvent porter une mutation ou une pluralité de mutations qui sont responsables d'une maladie génétique de la peau humaine.

Les "cellules souches pluripotentes induites" de la présente invention sont des cellules induites pour avoir une pluripotence en reprogrammant une cellule somatique par un procédé connu et similaire. Plus précisément, une cellule induite pour avoir une pluripotence peut être obtenue en reprogrammant des cellules somatiques différenciées telles que les fibroblastes, les cellules mononucléaires du sang périphérique et similaires par l'expression d'une combinaison d'une pluralité de gènes sélectionnés dans le groupe constitué par les gènes de reprogrammation, y compris Oct3 / 4, Sox2, On peut mentionner Klf4, Myc (c-Myc, N-Myc, L-Myc), Glis1, Nanog, Sall4, Iin28, Esrrb et similaires. Des exemples de combinaison préférable de facteurs de reprogrammation comprennent (1) Oct3 / 4, Sox2, Klf4 et Myc (c-Myc ou L-Myc) et (2) Oct3 / 4, Sox2, Klf4, Lin28 et L-Myc.

Des cellules souches pluripotentes induites ont été obtenues par Yamanaka et al. à partir d'une cellule de souris en 2006. En 2007, des cellules souches pluripotentes induites ont également été obtenues à partir de fibroblastes humains et celles-ci possèdent une compétence de pluripotence et d'auto-renouvellement similaire à celle des cellules souches embryonnaires.

Les termes "différenciation", tels qu'utilisés ici, désignent un processus par lequel une cellule non spécialisée ou relativement moins spécialisée devient relativement plus spécialisée. Dans le contexte de l'ontogenèse cellulaire, l'adjectif « différencié » est un terme relatif. Par conséquent, une "cellule différenciée" est une cellule qui a progressé plus loin dans une certaine voie de développement que la cellule avec laquelle elle est comparée. Une cellule relativement plus spécialisée peut différer d'une cellule non spécialisée ou relativement moins spécialisée par une ou plusieurs caractéristiques phénotypiques démontrables, telles que, par exemple, la présence, l'absence ou le niveau d'expression de composants ou produits cellulaires particuliers, par exemple, ARN, protéines ou d'autres substances, l'activité de certaines voies biochimiques, l'apparence morphologique, la capacité de prolifération et / ou la cinétique, le potentiel de différenciation et / ou la réponse aux signaux de différenciation, etc., où de telles caractéristiques signifient la progression de la différenciation vers la cellule relativement plus spécialisée. Le processus de différentiation à partir de cellules souches pluripotentes comprend généralement une première étape dite d'induction de la différentiation, au cours de la laquelle les cellules s'engagent dans une voie de différentiation pour donner des progéniteurs (à différents stades de différentiation), puis une deuxième étape de « maturation », au cours de laquelle la différentiation se poursuit, pour aboutir à une population de cellules complètement différenciées.

Selon un mode de réalisation de l'invention, les cellules souches pluripotentes sont des cellules hiPSC spécifiques d'un donneur, obtenues par reprogrammation de cellules mononucléaires de sang périphérique dudit donneur. Les protocoles de reprogrammation de cellules mononucléaires de sang périphérique du donneur ou de cellules CD34+ isolées de sang de cordon ombilical sont connus de l'homme de l'art.

Par « milieu adapté à la culture des fibroblastes » ou « milieu de culture des fibroblastes », on entend tout milieu qui contient les nutriments et facteurs permettant la culture *in vitro* de fibroblastes. Typiquement, un tel milieu peut comprendre des acides aminés, des minéraux et oligo-éléments (dont le sélénium, le manganèse et le zinc), des vitamines (du groupe B, notamment les folates, le niacinamide et la biotine), du glucose, du pyruvate, un tampon pH et des facteurs de croissance ou cofacteurs (tels que l'insuline, l'hydrocortisone, l'EGF (facteur de croissance épidermique) et le FGF (facteur de croissance des fibroblastes)).

De manière préférée, le milieu adapté à la culture des fibroblastes comprend de l'insuline, de l'hydrocortisone, de l'EGF et du FGF.

De manière préférée, le milieu adapté à la culture des fibroblastes est supplémenté en sérum de manière à obtenir une concentration finale en sérum de 5% (vol/vol). Typiquement, le sérum utilisé dans le procédé de l'invention est un sérum de grade clinique, approuvé BPF, le milieu complémenté avec ce sérum est appelé « Haute Certification » ou « HC ».

Un milieu adapté à la culture des fibroblastes particulièrement préféré est le milieu CnT-PR-F, commercialisé par la société CELLnTEC. Ce milieu a été particulièrement conçu pour la culture des fibroblastes. Il comprend classiquement des acides aminés essentiels, des minéraux et oligo-éléments (dont le sélénium, le manganèse et le zinc), des vitamines (du groupe B, notamment les folates, le niacinamide et la biotine), du glucose, du pyruvate, un tampon pH, mais contient en plus des milieux classiques de l'insuline, de l'hydrocortisone, et les facteurs de croissance EGF et FGF.

Dans un mode de réalisation de l'invention, le milieu adapté à la culture des fibroblastes n'est pas le milieu NHK (« Normal Human Keratinocyte medium »), décrit dans Hewitt et al. 2009 et dans l'exemple 2.

Dans un mode de réalisation de l'invention, le milieu adapté à la culture des fibroblastes n'est pas le milieu SCES (« Serum-containing Embryonic Stem cell medium »), décrit dans Hewitt et al. 2009 et dans l'exemple 2.

Hewitt K, Shamis Y, Carlson M, Aberdam E, Aberdam D, Garlick J. Three-dimensional epithelial tissues generated from human embryonic stem cells. Tissue Eng Part A. 2009 15(11) : 3417-3426.

Plus particulièrement, la présente invention concerne un procédé de préparation de fibroblastes dérivés de cellules souches pluripotentes humaines comprenant les étapes de:
(a) de manière optionnelle, former et cultiver des agrégats ou des amas desdites cellules souches pluripotentes sur un système adhérent pour soutenir la fixation et la croissance cellulaire en présence d'un milieu adapté à la culture de cellules souches pluripotentes humaines;
(b) cultiver les cellules souches pluripotentes humaines ou les agrégats ou amas adhérents desdites cellules souches pluripotentes sur une surface de culture cellulaire revêtue d'un revêtement de matrice protéique défini en présence d'un milieu à adapté à la culture des fibroblastes ;
(c) différenciation des cellules souches pluripotentes humaines en fibroblastes par culture sur une matrice protéique en présence d'un milieu adapté à la culture des fibroblastes, pendant 12 à 16 jours, de préférence 13 à 15 jours, de manière encore plus préférée 14 jours;
d) de manière optionnelle, maturation des fibroblastes obtenus à l'étape c) au moins pendant un passage sur une matrice protéique en présence d'un milieu adapté à la maturation ;
e) de manière optionnelle, maturation des fibroblastes obtenus à l'étape d) pendant un passage en présence d'un milieu adapté à la maturation, avec ou sans matrice protéique ;
e) sélectionner les cellules et obtenir une population homogène de fibroblastes.

De manière préférée, le milieu adapté à la maturation des fibroblastes peut être le même milieu que le milieu utilisé à l'étape c). De manière encore plus préférée, le milieu adapté à la maturation des fibroblastes est dépourvu de BMP-4.

Durant l'étape optionnelle a) du procédé selon l'invention, les cellules souches pluripotentes sont amplifiées afin d'obtenir une population suffisante pour les besoins expérimentaux ou cliniques. Au cours de cette étape d'amplification, les cellules forment des colonies. Le passage des cellules peut comprendre soit un passage manuel (qui consiste en un découpage en clumps ou morceau de colonies de façon manuel par l'opérateur à l'aide d'une aiguille), soit un passage enzymatique (par exemple à l'aide d'une solution d'EDTA). Par « passage » ou « passage cellulaire, on entend au sens de la présente invention toutes techniques de culture cellulaire bien connues de l'homme du métier. Par exemple, ce terme peut faire référence à une technique qui implique les étapes de (1) libération des cellules d'un support solide ou d'un substrat et dissociation de ces cellules, et (2) dilution des cellules dans un milieu approprié pour une prolifération cellulaire supplémentaire. Le passage des cellules peut également faire référence à l'élimination d'une partie du milieu liquide contenant des cellules cultivées et à l'ajout de milieu liquide au récipient de culture d'origine pour diluer les cellules et permettre une prolifération cellulaire supplémentaire. De plus, les cellules peuvent également être ajoutées à un nouveau récipient de culture qui a été supplémenté avec un milieu approprié pour une prolifération cellulaire supplémentaire.

Dans un mode de réalisation de l'invention, l'étape c) de différenciation a lieu pendant 12 à 16 jours, de préférence 13 à 15 jours, de manière encore plus préférée 14 jours.

Le milieu adapté à la culture des fibroblastes est supplémenté avec de la protéine morphogénétique osseuse 4 (« Bone Morphogenetic Protein 4 » ou « BMP-4 » pendant tout ou partie de l'étape b). Typiquement, la concentration finale en BMP-4 est d'environ 0,01 à 50 nM, de préférence environ 0,2 à 0,4 nM, de manière encore plus préférée environ 0,27nM.

La protéine BMP-4 est de préférence une protéine recombinante ayant la séquence de la forme humaine de BMP-4, par exemple la BMP-4 commercialisée par Peprotech sous la référence AF-120-05ET.

Selon un mode de réalisation, le milieu est supplémenté en BMP-4 de J1 à J6.

Cette supplémentation peut être réalisée par l'ajout de BMP-4 à J1, le milieu de culture n'étant pas renouvelé dans les jours suivants (un seul « pulse »).

Alternativement, la supplémentation peut être réalisée par l'ajout de BMP-4 à J1 et à J4 (deux « pulses »).

Typiquement, la BMP-4 n'est plus présente dans le milieu à partir de J7.

A l'issue de l'étape c), les fibroblastes ou précurseurs de fibroblastes obtenus sont isolés grâce à leurs propriétés d'adhérence afin de poursuivre leur maturation.

Selon un mode de réalisation de l'invention, les fibroblastes ou précurseurs de fibroblastes sont isolés par sélection « naturelle ». A l'issue de l'étape c), les cellules sont ensemencées à très forte densité (minimum 50 000 cellules/cm²) sur une matrice définie pour permettre aux fibroblastes différenciés d'adhérer et d'être cultivés avec une densité suffisante (minimum 10 000 cellules/cm²) permettant de limiter le stress cellulaire induit par la culture. Le lendemain, les cellules d'intérêt sont accrochées au substrat alors que les cellules mal ou non différenciées ne le sont pas.

La présente invention concerne également une population de fibroblastes, préférentiellement de fibroblastes de tissu conjonctif sous-jacents d'un épithélium, en particulier de fibroblastes dermiques, directement obtenue par le procédé décrit ci-dessus.

De manière avantageuse, la population de fibroblastes ainsi obtenue est homogène.

De manière avantageuse, la méthode selon l'invention permet d'obtenir préférentiellement des fibroblastes papillaires.

En effet, il existe dans le derme deux types de fibroblastes : les fibroblastes papillaires et les fibroblastes réticulaires. De manière avantageuse, le procédé selon la présente invention permet d'obtenir préférentiellement des fibroblastes papillaires. Ces fibroblastes sont plus prolifératifs et aident à la bonne stratification de l'épiderme. Ainsi, pour des applications de réparation du derme, il est intéressant d'avoir majoritairement des fibroblastes papillaires.

Par « fibroblastes papillaires » on entend une population de cellules exprimant majoritairement le marqueur des fibroblastes papillaires podoplanine (PDPN). Selon l'invention, on considère qu'une population de cellules exprime majoritairement un marqueur donné si au moins 90%, de préférence au moins 95%, de manière plus préférée encore au moins 98% des cellules expriment un marqueur donné. L'expression d'un marqueur donné par une population de cellules peut être mesurée par toute technique appropriée, telle que le FACS.

La présente invention concerne également l'utilisation de la population de fibroblastes dermiques pour la préparation d'un substitut de peau.

### Brève description des Figures

D'autres caractéristiques, détails et avantages de l'invention apparaîtront à la lecture des Figures annexées.
**Fig. 1**

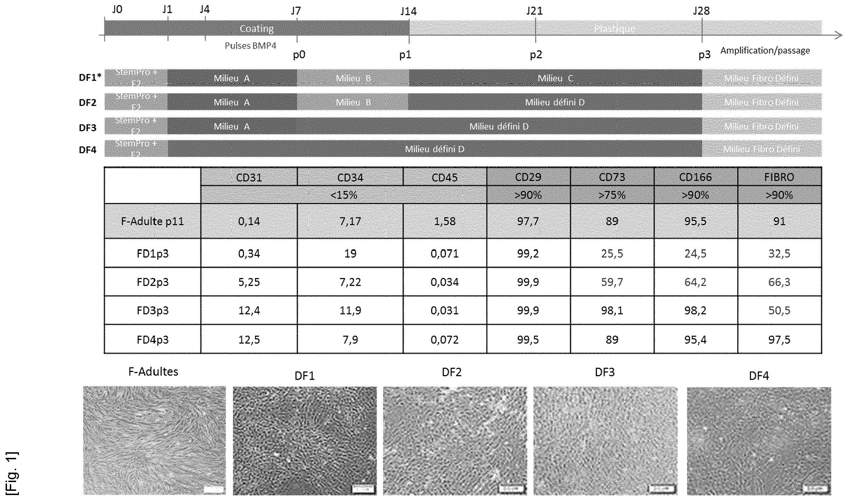

   [Fig. 1] représente les protocole de différenciation de cellules hESC en fibroblaste.
**Fig. 2**

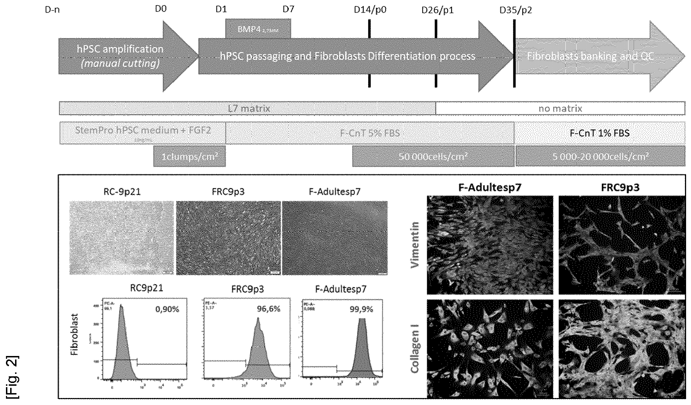

   [Fig. 2] représente le protocole de différenciation de cellules hESC en fibroblastes selon l'invention.
**Fig. 3**

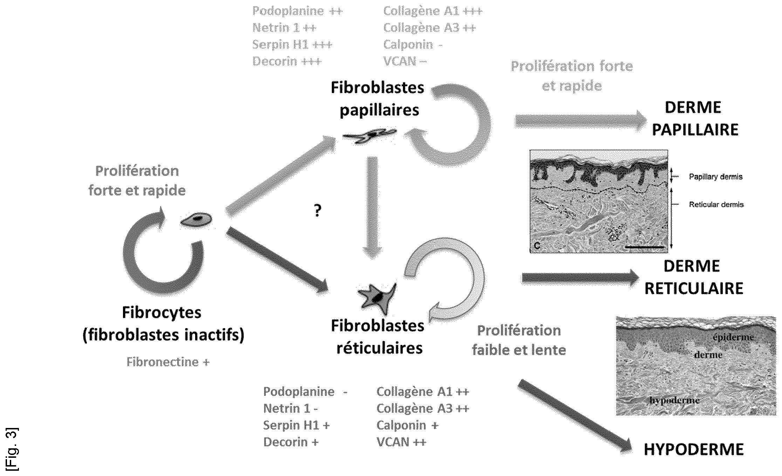

   [Fig. 3] représente les sous-types de fibroblastes dans la peau et les marqueurs associés.
**Fig. 4**

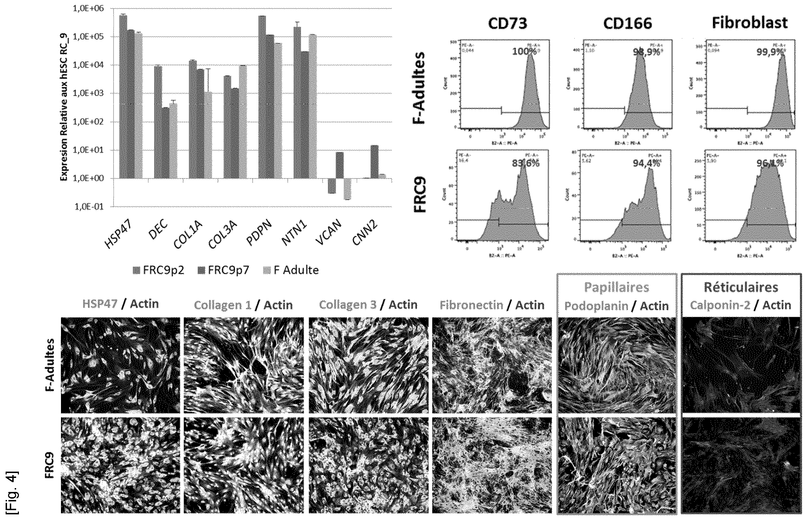

   [Fig. 4] représente le contrôle qualité des fibroblastes dérivés de hPSC.
**Fig. 5**

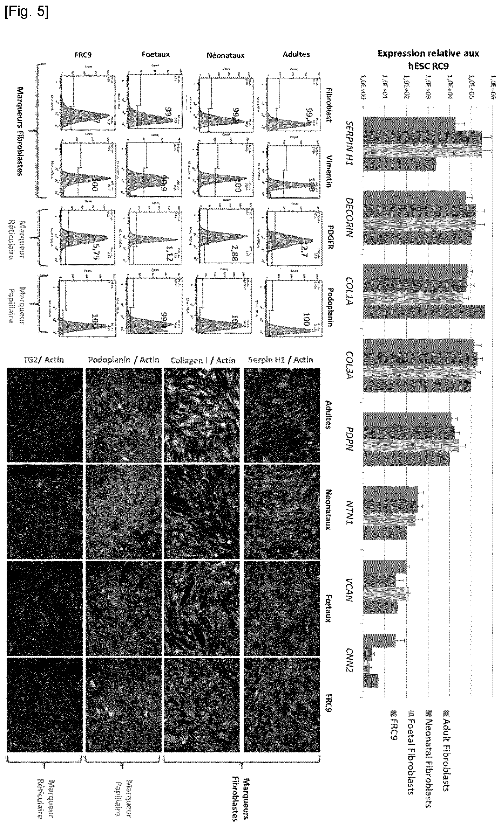

   [Fig. 5] représente la comparaison des FRC9 et des fibroblastes primaires.
**Fig. 6**

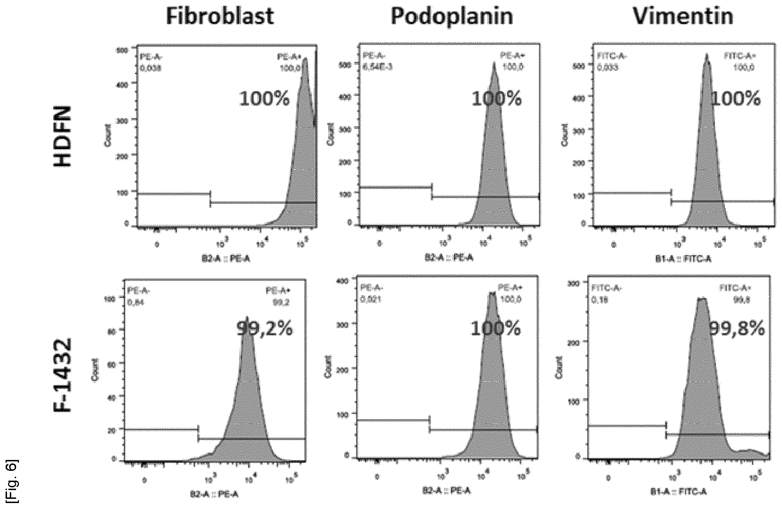

   [Fig. 6] représente la différenciation en fibroblastes à partir d'IPS des F-1432.

### Exemples

### Exemple 1 : Matériels et méthodes

Lignée de cellules pluripotentes utilisées : lignée d'hES (RC9, Roslin Cells)

### Culture des fibroblastes primaires :

Les différents types de fibroblastes primaires utilisés sont :
- Human Dermal Fibroblasts pooled foreskin: F-Adultes (HDFp, CELLnTEC)
- Normal Human Dermal Fibroblasts Fetal Foreskin: HDFF (Cell Application, 106-05f)
- Normal Human Dermal Fibroblasts neonatal Foreskin: HDFN (Promocell, C-12300)
- Normal Human Dermal Fibroblasts adult: HDFA (Promocell, C-12302)

### Protocole de différenciation de grade clinique selon l'invention :

Des colonies de cellules hPSC sont découpées à l'aiguille et 1 clumps/cm² est ensemencé dans les contenants coatés avec de la matrice L7 en milieu StemPro supplémenté en FGF-2 stabilisé pendant 24h (J0). Le lendemain le milieu de culture est changé par le milieu CnT-Prime-Fibroblast (F-CnT) supplémenté en sérum de veau fœtal défini et sécurisé (concentration finale : 5%) jusqu'à J14. Un traitement au BMP-4 à 0.27nM est appliqué à J1 et J4.

A partir de J14/p0, lorsque la confluence est atteinte, les cellules sont passées par traitement à la trypsine pendant 5min à +37°C. Les cellules en cours de différenciation sont réensemencées à 50 000 cellules par cm² jusqu'à J35/p2 sur matrice L7. Les cellules sont ainsi prêtes à être utilisées (banking, clinique, utilisation autre).

### Exemple 2 : Protocole de différenciation

Plusieurs conditions de différenciation ont été testées et comparées.

La condition 1 (FD1 ou DF1) est réalisée avec les mêmes milieux que le protocole de Shamis et al. jusqu'à p3/J28, mais sans feeder et sur la matrice L7.

Les milieux NHK et SCES sont décrits dans la publication Hewitt K, Shamis Y, Carlson M, Aberdam E, Aberdam D, Garlick J. Three-dimensional epithelial tissues generated from human embryonic stem cells. Tissue Eng Part A. 2009 15(11) : 3417-3426.

Le milieu NHK comprend un mélange DMEM :F12 3:1, FCII à 5%, adénine 0.18mM, HEPES 8mM, hydrocortisone 0.5 µg/mL, toxine cholérique 10⁻¹⁰M, EGF 10ng/mL et insuline 5 µg/mL.

Le milieu SCES comprend un mélange DMEM :F12 1 :1, FCII 5% et des acides aminés non-essentiels à 1%.

La condition 2 (DF2 ou FD2) est réalisée avec les mêmes milieux que le protocole original (DF1), c'est-à-dire le protocole de Shamis mais sans feeder et sur la matrice L7, jusqu'à p1/J14 puis le milieu F-CnT 5% jusqu'à p3/J28.

FD3 est réalisée avec les mêmes milieux que le protocole original jusqu'à p0/J7 puis le milieu F-CnT 5% jusqu'à p3/J28.

FD4 : est réalisée uniquement avec du milieu F-CnT 5% jusqu'à p3/J28.

A j21/p2, les cellules de toutes les conditions sont congelées et stockées en azote jusqu'à utilisation.

Pour réaliser les contrôles qualité, les cellules sont décongelées puis amplifiées en milieu F-CnT (sans ajout de 4% de sérum comme pour la différenciation) puis amplifiées jusqu'à 100% de confluence. La morphologie des cellules obtenues est similaire quelle que soit la condition de différenciation FD1, FD2, FD3 et FD4 mais différentes des cellules primaires (F-Adulte). Après le passage, les cellules sont analysées par FACS afin d'identifier les populations obtenues.

Plusieurs marqueurs sont analysés : les marqueurs de caractérisation fibroblastique : CD29 (intégrine β1), CD73/CD166 (marqueurs de cellules souches mésenchymateuses) et « Fibro » (marqueurs de fibroblastes).

L'analyse de l'expression de tous ces marqueurs sur les cellules primaires adultes (F-Adultes) montre que le profil « fibroblaste » se traduit par une expression de : CD29/CD166/FIBRO supérieure à 90% et CD73 supérieure à 75%.

L'analyse sur les 4 conditions de différenciation FD1, FD2, FD3 et FD4 montre qu'une seule différenciation atteint les spécifications obtenues avec les fibroblastes adultes : la condition FD4 avec une expression de CD29 à 99.5%, CD73 à 89%, CD166 à 95.4% et FIBRO à 97.5%.

Cette condition « FD4 » est donc validée pour le protocole de différenciation. Elle est également la plus simple car il n'y a plus qu'une seule référence de milieu pour l'ensemble du procédé à supplémenté à 5% de sérum pour l'étape « différenciation » entre J1 et la congélation à p2.

Pour les essais suivants, la densité a été remontée à 50 000 cellules/cm² en sortie de différenciation p0 et p1 puis à partir de p2, entre 5000 - 10 000 cellules/cm². A 50 000 cellules/cm², il y a plus de cellules adhérées qu'à 5000 - 10 000 cellules/cm² mais le niveau de cellules non adhérées est également supérieur. Cette étape de sortie de différenciation (p0) semble donc être une étape de sélection des « progéniteurs » de fibroblastes. Le tri se fait sur la base de l'adhésion : les cellules non adhérées ne sont pas suffisamment matures ou ne sont pas des progéniteurs de fibroblastes. Ceci permet donc de purifier les futurs fibroblastes et de rendre la population homogène au fur et à mesure des passages suivants.

Ces densités de sortie de différenciation et de passage ont permis d'obtenir des fibroblastes dérivés de hESC proliférant au moins jusqu'à p7 (passages suivants non testés) sans baisse notable des rendements à chaque passage.

Le protocole de différenciation établi se décompose donc en plusieurs étapes :
A J0, des colonies de cellules hPSC sont découpées à l'aiguille sous loupe binoculaire en clumps. Environ 1 clumps/cm² est ensemencé dans des contenants fermés par un bouchon (flasque) coatés avec le coating L7 et en milieu StemPro hESC avec 10ng/mL de FGF-2 stabilisé (conditions de culture des hPSC) pour permettre l'adhésion des hESC.

De J1 à J7, le milieu StemPro/FGF2 est remplacé par du milieu F-CnT 1% prêt à l'emploi qui est supplémenté avec 4% de SVF supplémentaire (pour avoir une concentration finale de 5%).

De J1 à J7, le milieu F-CnT 5% est supplémenté avec de la BMP-4 (0,273nM) pour inhiber la voie neurale. Deux pulses de BMP-4 sont effectués à J1 et à J4. La BMP-4 est présente de J1 à J7 inclus.

De J8 à J13, les cellules sont entretenues avec du milieu F-CnT 5% pour permettre la prolifération et la différenciation des hPSC.

A J14 (p0), les cellules rincées au PBS1X sont mises en contact avec de la trypsine EDTA 0,05% pendant 5 à 10min. L'action de la trypsine est stoppée avec du SVF à 10% et les cellules sont filtrées avec un tamis de 40µm pour éliminer les amas cellulaires non dissociés. Les cellules sont ensuite ensemencées à 50 000cellules/cm² dans des contenants fermés par un bouchon (flasque) coatés avec le coating L7 et en milieu F-CnT 5%.

La morphologie des FRC9 obtenus après le procédé de différenciation des hESC RC-9 est très proche des fibroblastes adultes (F-Adulte) et ne ressemble plus à celle des hESC RC-9 de départ. L'analyse de l'expression du marqueur FIBRO (ou Fibroblast) montre une absence d'expression de ce marqueur sur les hESC RC-9 et une expression de 96.6% sur les FRC9, proche du niveau des fibroblastes adultes à 99.9%. En plus de ce marqueur, les cellules expriment également la vimentine (marqueur de fibroblastes) et le collagène I (sécrété par les fibroblastes matures) au niveau du cytoplasme. Le collagène I ne semble pas être sécrété par les FRC9 ou les F-Adultes car il n'y a pas de dépôt. Pour observer la sécrétion de pro collagène I, des tests ELISA pourraient être réalisés.

### Exemple 3 : Caractérisation phénotypique des fibroblastes réticulaires et papillaires dérivés de hPSC

La littérature montre qu'il existe différents types de fibroblastes et qu'en fonction de leurs localisations au sein d'un même tissu ou dans un tissu différent, ils expriment des marqueurs spécifiques (Figure 3) :
Fibronectine : impliquée dans l'organisation de la matrice extracellulaire (MEC)
Serpin H1 (HSP47) : impliquée dans la synthèse des collagènes dans les fibroblastes dermiques
Décorine (DEC) : impliquée dans l'assemblage des fibrilles de collagènes
Collagène I et III (COL1A et COL3A) : protéines de la MEC
Podoplanine (PDPN) : impliquée dans le remodelage de la MEC par les fibroblastes papillaires
Calponin 2 (CNN2) : impliquée dans l'inhibition de la prolifération des fibroblastes réticulaires
Versican (VCAN) : protéine de la MEC sécrétée par les fibroblastes réticulaires

Un large panel de marqueurs tirés de la littérature a été testé en qPCR, en ICC et en FACS afin de déterminer l'identité exacte de la population de fibroblastes produite par le protocole de différenciation de la présente invention (Figure 4).

L'analyse de l'expression des gènes par qPCR (normalisation sur le gène 18S et sur les cellules RC-9) montre que les fibroblastes dérivés de RC9 expriment les marqueurs HSP47, DEC, COL1A, COLA3, PDPN et NTN1 à des niveaux forts similaires aux niveaux des fibroblastes adultes. Les marqueurs réticulaires VCAN et CNN2 ne sont pas ou peu exprimés par les FRC9p2 et les fibroblastes adultes. Une légère hausse de l'expression des marqueurs réticulaires VCAN et CNN2 sur les FRC9p7 est observée.

L'analyse par FACS montre que les FRC9 et les F-Adultes expriment les marqueurs CD73/CD166 à plus de 80% et le marquage « Fibroblast » à plus de 96%.

L'analyse par ICC, montre que les FRC9 et les F-Adultes ont des profils similaires :
L'HSP47 est bien exprimée au niveau du réticulum endoplasmique,
Les collagènes I et III sont également exprimés dans le cytoplasme avec une localisation proche du réticulum endoplasmique,
La fibronectine est exprimée dans le cytoplasme et il y a présence de dépôts à l'extérieur des cellules ce qui montre que la protéine a été sécrétée,
La podoplanine est exprimée dans l'ensemble du cytoplasme,
La calponine 2 est exprimée par très peu de cellules.

Les résultats montrent que le protocole de différenciation fibroblastique permet d'obtenir des fibroblastes dermiques de par l'expression de la serpinH1/HSP47 et que la population obtenue est majoritairement papillaire du fait de l'expression forte de la podoplanine et l'expression faible de la calponine 2 qui n'est observée que sur quelques cellules. Les F-Adultes et les FRC9 expriment également les collagènes I/III mais ces protéines ne semblent pas être sécrétées car aucun dépôt n'est détecté contrairement à la fibronectine qui est exprimée et sécrétée. La sécrétion du collagène par les F-Adultes et les FRC9 semblent nécessiter des conditions de culture différentes.

### Exemple 4 : Comparaison des fibroblastes dérivés de hPSC et de fibroblastes primaires

Toujours dans le but de caractériser les cellules et d'améliorer leurs contrôles qualité, une étude de comparaison entre les FRC9 et 3 lignées de fibroblastes primaires (fœtaux, néonataux et adultes) a été réalisée (Figure 5).

L'analyse par qPCR montre que le profil d'expression des gènes DECORIN, COL1A, COL3A, PDPN, NTN1 et VCAN est similaire entre les 3 types de fibroblastes primaires et les FRC9. L'expression du gène SERPIN H1 est proche des cellules adultes mais plus faible que celle des fibroblastes néonataux et fœtaux. Le gène CNN2 est faiblement exprimé par les FRC9 et les fibroblastes adultes et n'est pas exprimé dans les fibroblastes néonataux et fœtaux.

L'analyse par FACS montre que les fibroblastes adultes, néonataux et FRC9 ont un profil similaire pour les marqueurs « fibroblast », vimentine et podoplanine avec une expression de plus de 97%. Les fibroblastes fœtaux ont une expression plus faible pour le marquage vimentine avec 90,9%. Pour le marquage PDGFR (marquage des cellules réticulaires, impliqué dans la différenciation cellulaire), on observe que globalement le profil est faiblement exprimé avec une expression inférieure à 13% pour toutes les lignées.

L'analyse par ICC montrent que les 3 types de fibroblastes primaires et les FRC9 expriment fortement et de façon homogène, les marqueurs Serpin H1, Collagène I, podoplanin avec une localisation au réticulum endoplasmique pour les marquages Serpin H1 et Collagène I. Le marqueur TG2 (Transglutaminase 2, impliquée dans la structure de la MEC), est très peu exprimé par les 4 types de fibroblastes.

Les analyses réalisées avec les 3 types de fibroblastes ne permettent pas de les différencier et donc d'appliquer un profil « adulte », « néonatal » ou « fœtal » aux FRC9.

### Exemple 5 : Différenciation de la lignée iPSC PC1432 en fibroblastes

Le procédé utilisé pour la lignée RC 9 fonctionne également pour les iPS.

L'analyse FACS des iPS montre un profil d'expression similaire aux HDNF avec plus de 99% d'expression des marqueurs « Fibroblast », podoplanin et vimentin.

Le procédé de différenciation permet donc d'obtenir des fibroblastes à partir d'iPS.

## Revendications

1. Procédé de différenciation en fibroblastes à partir de cellules souches pluripotentes humaines, **caractérisé en ce que** les cellules souches pluripotentes humaines sont cultivées sur un système adhérent en présence d'un milieu adapté à la culture des fibroblastes et en l'absence de cellules nourricières.

2. Procédé selon la revendication 1 **caractérisé en ce que** les fibroblastes sont des fibroblastes dermiques.

3. Procédé selon l'une quelconque des revendications 1 ou 2, **caractérisé en ce que** les fibroblastes sont majoritairement des fibroblastes papillaires.

4. Procédé selon l'une quelconque des revendications 1 à 3 **caractérisé en ce que** le milieu adapté à la culture des fibroblastes comprend de l'insuline, de l'hydrocortisone, du facteur de croissance épidermique (EGF) et du facteur de croissance des fibroblastes (FGF).

5. Procédé selon l'une quelconque des revendications ci-dessus, **caractérisé en ce que** le milieu adapté à la culture des fibroblastes est supplémenté en Bone Morphogenetic Protein 4 (BMP-4).

6. Procédé selon l'une quelconque des revendications précédentes, **caractérisé en ce qu'**il comprend les étapes de :
(a) de manière optionnelle, former et cultiver des agrégats ou des amas desdites cellules souches pluripotentes sur un système adhérent pour soutenir la fixation et la croissance cellulaire en présence d'un milieu adapté à la culture de cellules souches pluripotentes humaines;
(b) cultiver les cellules souches pluripotentes humaines ou les agrégats ou amas adhérents desdites cellules souches pluripotentes sur une surface de culture cellulaire revêtue d'un revêtement de matrice protéique défini en présence d'un milieu à adapté à la culture des fibroblastes ;
(c) différenciation des cellules souches pluripotentes humaines en fibroblastes par culture sur une matrice protéique en présence d'un milieu adapté à la culture des fibroblastes, pendant 12 à 16 jours, de préférence 13 à 15 jours, de manière encore plus préférée 14 jours;
d) de manière optionnelle, maturation des fibroblastes obtenus à l'étape c) au moins pendant un passage sur une matrice protéique en présence d'un milieu adapté à la maturation ;
e) de manière optionnelle, maturation des fibroblastes obtenus à l'étape d) pendant un passage en présence d'un milieu adapté à la maturation, avec ou sans matrice protéique ;
e) sélectionner les cellules et obtenir une population homogène de fibroblastes.

7. Procédé selon la revendication 6 dans lequel l'étape b) d'amplification des cellules souches comprend le passage manuel des cellules souches pluripotentes.

8. Procédé selon la revendication 6 dans lequel l'étape b) d'amplification des cellules souches pluripotentes comprend le passage enzymatique des cellules souches pluripotentes.

9. Procédé selon l'une quelconque des revendications 6 à 7 dans lequel la protéine morphogénétique osseuse 4 (Bone Morphogenetic Protein-4 ou BMP-4) est ajoutée à une concentration comprise entre 0,1 et 0,5 nM, de préférence entre 0,2 et 0,4 nM, de manière encore plus préférée environ 0,27nM, à J1 et J4.

10. Procédé de préparation d'un tissu conjonctif, de préférence un derme, préférentiellement une peau 3D, comprenant des fibroblastes obtenus selon le procédé de l'une quelconque des revendications 1 à 9.

11. Utilisation des fibroblastes obtenus selon le procédé de l'une quelconque des revendications 1 à 9 en culture cellulaire pour l'évaluation de composés.

12. Utilisation des fibroblastes obtenus selon le procédé de l'une quelconque des revendications 1 à 9 en culture cellulaire pour la production de protéines à usage thérapeutique.
